(19) Europäisches Patentamt
European Patent Office
Office européen des brevets

(11) Veröffentlichungsnummer : **0 110 041**
**B1**

# (12) EUROPÄISCHE PATENTSCHRIFT

(45) Veröffentlichungstag der Patentschrift : **29.07.87**

(51) Int. Cl.⁴ : **C 07 J 5/00**, C 07 J 7/00, A 61 K 31/57

(21) Anmeldenummer : **83109308.3**

(22) Anmeldetag : **20.09.83**

## (54) Neue 6alpha-Methylkortikoide, ihre Herstellung und Verwendung.

(30) Priorität : **22.11.82 DE 3243482**

(43) Veröffentlichungstag der Anmeldung : **13.06.84 Patentblatt 84/24**

(45) Bekanntmachung des Hinweises auf die Patenterteilung : **29.07.87 Patentblatt 87/31**

(84) Benannte Vertragsstaaten : **AT BE CH DE FR GB IT LI LU NL SE**

(56) Entgegenhaltungen :
**EP-A- 0 054 786**
**DE-B- 1 443 957**
**GB-A- 921 533**
**US-A- 3 053 832**
**JOURNAL OF THE AMERICAN CHEMICAL SOCIETY, Band 77, Nr. 15, 5. August 1955, Seiten 4181-4182, Washington, D.C., USA J. FRIED et al.: "Synthesis and biological activity of 1 - and 6-dehydro-9alpha-halocorticoids"**
**JOURNAL OF THE AMERICAN CHEMICAL SOCIETY, Band 75, Nr. 9, 5. Mai 1953, Seiten 2273-2274, Washington, D.C., USA J. FRIED et al.: "Synthesis of 17alpha-hydroxycorticosterone and its 9alpha-halo derivatives from 11-EPI-17alpha-hydroxycorticosterone"**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen
Müllerstrasse 170/178 Postfach 65 03 11
D-1000 Berlin 65 (DE)**

(72) Erfinder : **Annen, Klaus, Dr.
Seegefelderstrasse 194
D-1000 Berlin 20 (DE)**
Erfinder : **Laurent, Henry, Dr.
Glambecker Weg 21
D-1000 Berlin 28 (DE)**
Erfinder : **Hofmeister, Helmut, Dr.
Weislingenstrasse 4
D-1000 Berlin 28 (DE)**
Erfinder : **Wiechert, Rudolf, Prof. Dr.
Petzowerstrasse 8 A
D-1000 Berlin 39 (DE)**
Erfinder : **Wendt, Hans, Dr.
Ernst-Ring-Strasse 14
D-1000 Berlin 38 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 110 041 B1

Jouve, 18, rue St-Denis, 75001 Paris, France

**Beschreibung**

Die Erfindung betrifft
6α-Methylkortikoide del allgemeinen Formel I

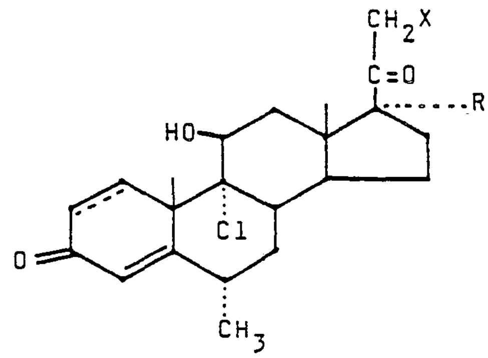

(I)

worin ..... eine Einfachbindung oder eine Doppelbindung bedeutet,
R eine Acyloxygruppe mit maximal 8 Kohlenstoffatomen und
X ein Chloratom, eine Hydroxygruppe oder eine Acyloxygruppe mit maximal 8 Kohlenstoffatomen darstellen.

Die neuen 6α-Methylkortikoide der allgemeinen Formel I können als Acyloxygruppen R aliphatische, cycloaliphatische oder aromatische Reste wie beispielsweise eine Acetylgruppe, eine Propionylgruppe, eine Butyrylgruppe, eine Isobutyrylgruppe, eine Valerylgruppe, eine 3-Methylbutyrylgruppe, eine Trimethylacetylgruppe eine Hexanoylgruppe oder Benzoylgruppe tragen.

Es wurde gefunden, daß die anmeldungsgemäßen 6α-Methylkortikoide überraschenderweise oft bei topischer Applikation eine signifikant stärkere Wirksamkeit besitzen als die vorbekannten 6α-Methylkortikoide « (Siehe z. B. Ep-A-54 786 oder US-A-3 053 832) ». Diese Wirksamkeit ist oft sogar noch signifikant stärker als diejenige difluorierter « Edelkortikoide », wie etwa das 6α, 9α-Difluor-11β-hydroxy-16α-methyl-21-valeryloxy-1,4-pregnadien-3,20-dion (= Nerisona).

Bei systemischer Applikation sind diese 6α-Methylkortikoide überraschenderweise oft schwächer wirksam als die entsprechenden vorbekannten 6α-Methylkortikoide.

Die neuen 6α-Methylkortikoide der allgemeinen Formel I eigenen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus and ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen 6α-Methylkortikoide können nach einem Verfahren hergestellt werden, welches unter den Bedingungen durchgeführt wird, wie sie in den deutschen Patentanmeldungen Nr. 26 45 104, 26 45 105, 23 40 591 und 19 58 549, im US-Patent Nr. 3,383,394 sowie in J. Org. Chem. 38, 1973, 4203 beschrieben sind.

Das Verfahren ist dadurch gekennzeichnet, daß man in an sich bekannter Weise

a) ein 6α-Methylkortikoid der allgemeinen Formel II

(Siehe Formel Seite 3 f.)

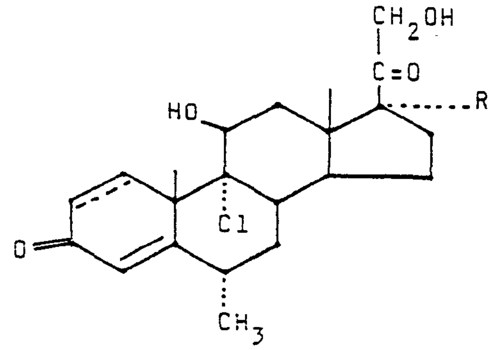

(II)

worin ..... und R die obengenannte Bedeutung besitzen, in der 21-Position verestert oder chloriert, oder

b) ein 6α-Methylkortikoid der allgemeinen Formel III

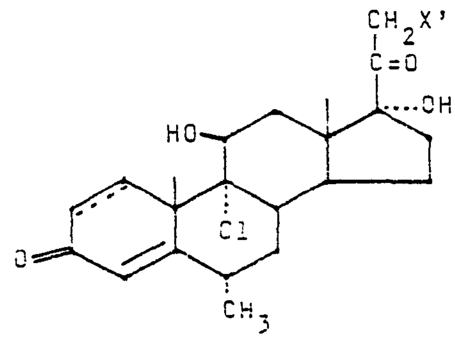

(III)

worin ..... und X' die obengenannte Bedeutung besitzen, in der 11-Position mit einer Trialkylsilylverbindung veräthert oder mit einem Derivat einer stark aciden Monocarbonsäure verestert, anschließend die 17-Position mit einem Carbonsäurechlorid oder Carbonsäureanhydrid in Gegenwart von 4-Dimethylaminopyridin acyliert und die Schutzgruppe in der 11-Position abspaltet,

oder

c) an die $\Delta^{9(11)}$-Doppelbindung eines 6α-Methylkortikoids der allgemeinen Formel IV

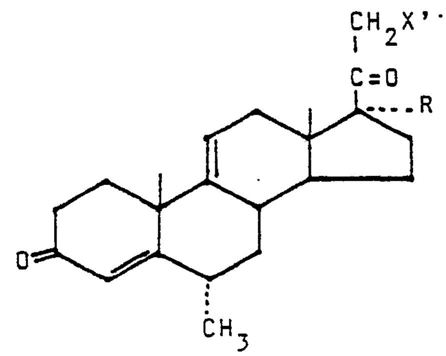

(IV)

worin ....., X' und R die obengenannte Bedeutung besitzen, unterchlorige Säure anlagert oder daß man in an sich bekannter Weise die Ortoestergruppierung eines Kortikoids der allgemeinen Formel V

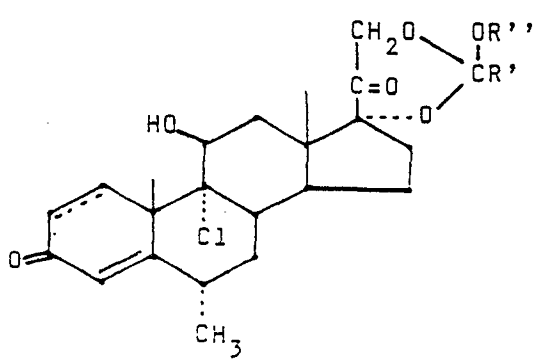

(V)

worin ..... und R' die obengenannte Bedeutung besitzen und R" einen Alkylrest mit 1 bis 4 Kohlenstoffatomen darstellt hydrolytisch oder mit Trimethylchlorsilan spaltet.

Die nachfolgenden Ausführungsbeispiele dienen zur Erläuterung des erfindungsgemäßen Verfahrens.

Beispiel 1

a) Aus einer Lösung von 5.0 g 9α-Chlor-11β.17α.21-trihydroxy-6α-methyl-1.4-pregnadien-3.20-dion und 0.5 g Pyridiniumtosylat in 35 ml Dimethylformamid und 350 ml Benzol werden bei 130 °C über einen Wasserabscheider 150 ml Benzol abdestilliert. In die heiße Reaktionslösung läßt man 10 ml Orthoessigsäuretriethylester langsam zulaufen und destilliert anschließend weiter Benzol und andere leichtflüchtige Reaktionskomponenten ab. Man fügt nun 4 ml Pyridin hinzu und engt i.Vak. zur Trockne ein. Es wird 9α-Chlor-17α.21-(ethoxyethylidendioxy)-11β-hydroxy-6α-methyl-1.4-pregnadien-3.20-dion als Öl isoliert.

b) Das rohe 9α-Chlor-17α.21-(ethoxyethylidendioxy)-11β-hydroxy-6α-methyl-1.4-pregnadien-3.20-dion wird in 150 ml Methanol gelöst und mit einem Gemisch aus 54 ml 0.1N Essigsäure und 6 ml 0.1M wäßriger Natriumacetat-Lösung 1 h bei 100 °C Badtemperatur gerührt. Man engt die Lösung auf 1/3 ihres Volumens ein, gibt auf Wasser und wäscht die Essigesterextrakte neutral. Nach dem Trocknen und Einengen wird das Rohprodukt an 500 g Kieselgel mit einem Methylenchlorid-Aceton-Gradienten (0-20 % Aceton) gereinigt. Man erhält 4.6 g 17α-Acetoxy-9α-chlor-11β.21-dihydroxy-6α-methyl-1.4-pregnadien-3.20-dion vom Schmp. 216-218 °C.

Beispiel 2

a) Analog Beispiel 1a) werden 2.0 g 9α-Chlor-11β.17α.21-trihydroxy-6α-methyl-1.4-pregnadien-3.20-dion mit Orthopropionsäuretriethylester zum 9α-Chlor-17α.21-(ethoxypropylidendioxy)-11β-hydroxy-6α-methyl-1.4-pregnadien-3.20-dion als Öl umgesetzt.

b) Das rohe 9α-Chlor-17α.21-(ethoxypropylidendioxy)-11β-hydroxy-6α-methyl-1.4-pregnadien-3.20-dion wird analog Beispiel 1b) umgesetzt, aufgearbeitet und gereinigt. Man isoliert 1.6 g 9α-Chlor-11β.21-dihydroxy-6α-methyl-17α-propionyloxy-1.4-pregnadien-3.20-dion vom Schmp. 201-203 °C.

Beispiel 3

a) Man tropft zu einer Lösung von 12.8 g 21-Acetoxy-11β-hydroxy-6α-methyl-17α-propionyloxy-1.4-pregnadien-3.20-dion in 65 ml Dimethylformamid und 15 ml Pyridin bei Raumtemperatur 8 ml Methansulfonsäurechlorid und rührt anschließend 2 h bei 80 °C Badtemperatur weiter. Nach der Eiswasserfällung und üblichen Aufarbeitung reinigt man das Rohprodukt an 800 g Kieselgel mit einem Methylenchlorid-Aceton-Gradienten (0-8 % Aceton). Man erhält 7.0 g 21-Acetoxy-6α-methyl-17α-propionyloxy-1.4.9(11)-pregnatrien-3.20-dion vom Schmp. 185-187 °C.

b) 2.0 g 21-Acetoxy-6α-methyl-17α-propionyloxy-1.4.9(11)-pregnatrien-3.20-dion werden in 20 ml Dioxan gelöst und mit 1.8 g N-Chlor-succinimid versetzt. Nach dem Zutropfen von 10 ml einer 10 proz. Perchlorsäure-Lösung wird 5 h bei Raumtemperatur gerührt. Man gibt auf Eiswasser und arbeitet wie üblich auf. Das Rohprodukt wird an 200 g Kieselgel mit einem Methylenchlorid-Aceton-Gradienten (0-12 % Aceton) gereinigt. Ausbeute 1.6 g 21-Acetoxy-9α-chlor-11β-hydroxy-6α-methyl-17α-propionyloxy-1.4-pregnadien-3.20-dion vom Schmp. 232-233 °C.

Beispiel 4

a) Eine Lösung von 2.7 g Tristriphenylphosphin-rhodium-I-chlorid in 75 ml Methanol und 225 ml Benzol wird 1 h bei Raumtemperatur vorhydriert. Nach Zugabe von 3.0 g 21-Acetoxy-6α-methyl-17α-propionyloxy-1.4.9(11)-pregnatrien-3.20-dion wird 6.5 h weiterhydriert. Man engt zur Trockne ein und reinigt den Rückstand an 350 g Kieselgel mit einem Hexan-Essigester-Gradienten (0-40 % Essigester). Man erhält 2.5 g 21-Acetoxy-6α-methyl-17α-propionyloxy-4.9(11)-pregnadien-3.20-dion vom Schmp. 149-151 °C.

b) Unter den Bedingungen des Beispiels 3b) werden 2.0 g 21-Acetoxy-6α-methyl-17α-propionyloxy-4.9(11)-pregnadien-3.20-dion mit N-Chlorsuccinimid umgesetzt, aufgearbeitet und gereinigt. Man isoliert 1.05 g 21-Acetoxy-9α-chlor-11β-hydroxy-6α-methyl-17α-propionyloxy-4-pregnen-3.20-dion vom Schmelzp. 205-206 °C.

Beispiel 5

a) 5.0 g 17α.21-Dihydroxy-6α-methyl-1.4.9(11)-pregnatrien-3.20-dion werden analog Beispiel 1a) mit 10 ml Orthopropionsäuretriethylester zu 17α.21-(Ethoxypropylidendioxy)-6α-methyl-1.4.9(11)-pregnatrien-3.20-dion als Öl umgesetzt.

b) Das rohe 17α.21-(Ethoxypropylidendioxy)-6α-methyl-1.4.9(11)-pregnatrien-3.20-dion wird in 250 ml Dimethylformamid und 5 ml Trimethylchlorsilan 20 h bei 80 °C Badtemperatur gerührt. Man engt zur Trockne ein und reinigt das Rohprodukt an 600 g Kieselgel mit einem Methylenchlorid-Aceton-Gradienten (0-12 % Aceton). Ausbeute 3.5 g 21-Chlor-6α-methyl-17α-propionyloxy-1.4.9(11)-

pregnatrien-3.20-dion.

c) Analog Beispiel 3b) werden 1.5 g 21-Chlor-6α-methyl-17α-propionyloxy-1.4.9(11)-pregnatrien-3.20-dion mit N-Chlorsuccinimid umgesetzt, aufgearbeitet und gereinigt. Man isoliert 1.32 g 9α.21-Dichlor-11β-hydroxy-6α-methyl-17α-propionyloxy-1.4-pregnadien-3.20-dion. Schmp. 239-241 °C.

Beispiel 6

a) Wie in Beispiel 1a) beschrieben, werden 2.0 g 17α.21-Dihydroxy-6α-methyl1.4.9(11)-pregnatrien-3.20-dion mit Orthobuttersäuretriethylester zum 17α.21-(Ethoxybutylidendioxy)-6α-methyl-1.4.9(11)-pregnatrien-3.20-dion als Öl umgesetzt.

b) Das rohe 17α.21-(Ethoxybutylidendioxy)-6α-methyl-1.4.9(11)-pregnatrien-3.20-dion wird unter den Bedingungen des Beispiels 1b) mit einem Gemisch aus 0.1N Essigsäure und 0.1M Natriumacetat-Lösung behandelt, aufgearbeitet und gereinigt. Man erhält 1.5 g 17α-Butyryloxy-21-hydroxy-6α-methyl-1.4.9(11)-pregnatrien-3.20-dion.

c) Eine Lösung von 1.0 g 17α-Butyryloxy-21-hydroxy-6α-methyl-1.4.9(11)-pregnatrien-3.20-dion in 10 ml Pyridin wird mit 5.0 ml Essigsäureanhydrid 1 h bei Raumtemperatur gerührt und anschließend wie üblich aufgearbeitet. Nach dem Umkristallisieren aus Aceton/Hexan isoliert man 930 mg 21-Acetoxy-17α-butyryloxy-6α-methyl-1.4.9(11)-pregnatrien-3.20-dion.

d) Unter den Bedingungen des Beispiels 3b) werden 800 mg 21-Acetoxy-17α-butyryloxy-6α-methyl-1.4.9(11)-pregnatrien-3.20-dion mit N-Chlorsuccinimid umgesetzt, aufgearbeitet und gereinigt. Ausbeute 650 mg 21-Acetoxy-17α-butyryloxy-9α-chlor-11β-hydroxy-6α-methyl-1.4-pregnadien-3.20-dion. Schmp. 227-229 °C.

Beispiel 7

a) Zu einer Suspension von 12.2 g Kupfer(I)-jodid in 240 ml wasserfreiem Tetrahydrofuran tropft man bei 0 °C unter Argon 60 ml einer 1.6M Lithiummethyl-Lösung. Man rührt 15 min bei 0 °C weiter und kühlt die gelbliche Lösung auf —30 °C ab. Nach tropfenweiser Zugabe einer Lösung von 9.6 g 17α-Hydroxy-6α-methyl-21-valeryloxy-1.4.9(11)-pregnatrien-3.20-dion wird das Reaktionsgemisch 40 min bei —30 °C nachgerührt, anschließend auf eine eiskalte gesättigte Ammoniumchlorid-Lösung gegeben und mit Essigester extrahiert. Die organischen Extrakte werden wie üblich aufgearbeitet und das Rohprodukt an 100 g Kieselgel mit einem Methylenchlorid-Aceton-Gradienten (0-12 % Aceton) gereinigt. Ausbeute 7.3 g 21-Hydroxy-6α-methyl-17α-valeryloxy-1.4.9(11)-pregnatrien-3.20-dion.

b) Analog Beispiel 6c) werden 5.0 g 21-Hydroxy-6α-methyl-17α-valeryloxy-1.4.9(11)-pregnatrien-3.20-dion mit Propionsäureanhydrid umgesetzt, aufgearbeitet und gereinigt. Man isoliert 4.6 g 6α-Methyl-21-propionyloxy-17α-valeryloxy-1.4.9(11)-pregnatrien-3.20-dion.

c) Unter den Bedingungen des Beispiels 3b) werden 4.0 g 6α-Methyl-21-propionyloxy-17α-valeryloxy-1.4.9(11)-pregnatrien-3.20-dion mit N-Chlorsuccinimid umgesetzt, aufgearbeitet und gereinigt. Ausbeute 2.9 g 9α-Chlor-11β-hydroxy-6α-methyl-21-propionyloxy-17α-valeryloxy-1.4-pregnadien-3.20-dion. Schmp. 257-259 °C.

Beispiel 8

a) Wie im Beispiel 1a) beschrieben, werden 5.0 g 17α.21-Dihydroxy-6α-methyl-1.4.9(11)-pregnatrien-3.20-dion mit 10 ml Orthobenzoesäuretriethylester zu 17α.21-(Ethoxybenzylidendioxy)-6α-methyl-1.4.9(11)-pregnatrien-3.20-dion umgesetzt und aufgearbeitet.

b) Das rohe 17α.21-(Ethoxybenzylidenoxy)-6α-methyl-1.4.9(11)-pregnatrien-3.20-dion wird unter den Bedingungen des Beispiels 5b) mit Trimethylchlorsilan umgesetzt, aufgearbeitet und gereinigt. Man isoliert 3.2 g 17α-Benzoyloxy-21-chlor-6α-methyl-1.4.9(11)-pregnatrien-3.20-dion.

c) Analog Beispiel 3b) wird 1.0 g 17α-Benzoyloxy-21-chlor-6α-methyl-1.4.9(11)-pregnatrien-3.20-dion mit N-Chlorsuccinimid umgesetzt, aufgearbeitet und gereinigt. Ausbeute 920 mg 17α-Benzoyloxy-9α.21-dichlor-11β-hydroxy-6α-methyl-1.4-pregnadien-3.20-dion. Schmp. 230-232 °C.

Beispiel 9

a) Eine Lösung von 6.5 g 21-Acetoxy-17α-hydroxy-6α-methyl-1.4.9(11)-pregnatrien-3.20-dion in 80 ml Diethylglykoldimethylether wird mit 20.0 g 4-Dimethylaminopyridin und 20.0 ml Pivalinsäureanhydrid 5 d bei 80 °C Badtemperatur gerührt. Nach der Eiswasserfällung wird wie üblich aufgearbeitet und das Rohprodukt an 750 g Kieselgel mit einem Methylenchlorid-Aceton-Gradienten (0-8 % Aceton) gereinigt. Man isoliert 4.6 g 21-Acetoxy-6α-methyl-17α-trimethylacetoxy-1.4.9(11)-pregnatrien-3.20-dion. Schmp. 238-240 °C.

b) Unter den Bedingungen des Beispiels 3b) werden 2.0 g 21-Acetoxy-6α-methyl-17α-trimethylacetoxy-1.4.9(11)-pregnatrien-3.20-dion mit N-Chlorsuccinimid umgesetzt, aufgearbeitet und chromatographiert. Ausbeute 1.56 g 21-Acetoxy-9α-chlor-11β-hydroxy-6α-methyl-17α-trimethylacetoxy-1.4-pregnadien-3.20-dion. Schmp. 264-265 °C.

Beispiel 10

a) 1.8 g Tristriphenylphosphin-rhodium-I-chlorid werden in 50 ml MeOH und 150 ml Tetrahydrofuran 1 h vorhydriert. Nach Zugabe von 2.0 g 21-Acetoxy-6α-methyl-17α-trimethylacetoxy-1.4.9(11)-pregnatrien-3.20-dion wird 5.5 h weiterhydriert und anschließend zur Trockne eingeengt. Der Rückstand wird an 300 g Kieselgel mit einem Hexan-Essigester-Gradienten (0-50 % Essigester) gereinigt. Man isoliert 1.5 g 21-Acetoxy-6α-methyl-17α-trimethylacetoxy-4.9(11)-pregnadien-3.20-dion. Schmp. 162-163 °C.

b) 1.3 g 21-Acetoxy-6α-methyl-17α-trimethylacetoxy-4.9(11)-pregnadien-3.20-dion werden unter den Bedingungen des Beispiels 3b) umgesetzt, aufgearbeitet und gereinigt. Ausbeute 560 mg 21-Acetoxy-9α-chlor-11β-hydroxy-6α-methyl-17α-trimethylacetoxy-4-pregnen-3.20-dion. Schmp. 235-237 °C.

**Patentansprüche**

1. 6α-Methylkortikoide der allgemeinen Formel I

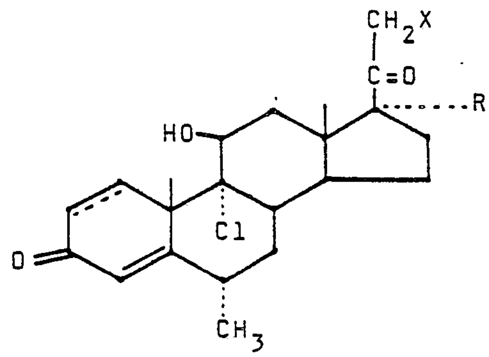

(I)

worin

..... eine Einfachbindung oder eine Doppelbindung bedeutet,
R eine Acyloxygruppe mit maximal 8 Kohlenstoffatomen und
X ein Chloratom, eine Hydroxygruppe oder eine Acyloxygruppe mit maximal 8 Kohlenstoffatomen darstellen.

2. 17α-Acetoxy-9α-chlor-11β,21-dihydroxy-6α-methyl-1,4-pregnadien-3,20-dion.
3. 9α-Chlor-11β,21-dihydroxy-6α-methyl-17α-propionyloxy-1,4-pregnadien-3,20-dion.
4. 21-Acetoxy-9α-chlor-11β-hydroxy-6α-methyl-17α-propionyloxy-4-pregnen-3,20-dion.
5. 9α,21-Dichlor-11β-hydroxy-6α-methyl-17α-propionyloxy-1,4-pregandien-3,20-dion.
6. 17α-Benzoyloxy-9α,21-dichlor-11β-hydroxy-6α-methyl-1,4-pregnadien-3,20-dion.
7. 9α-Chlor-11β-hydroxy-6α-methyl-21-propionyloxy-17-valeryloxy-1,4-pregnadien-3,20-dion.
8. 21-Acetoxy-9α-chlor-11β-hydroxy-6α-methyl-17α-trimethylacetoxy-4-pregnen-3,20-dion.
9. 21-Acetoxy-9α-chlor-11β-hydroxy-6α-methyl-17-trimethylacetoxy-1,4-pregnadien-3,20-dion.
10. 21-Acetoxy-9α-chlor-11β-hydroxy-6α-methyl-17-propionyloxy-1,4-pregnadien-3,20-dion.
11. 21-Acetoxy-17-butyryloxy-9α-chlor-11β-hydroxy-6α-methyl-1,4-pregnadien-3,20-dion.
12. Pharmazeutische Präparate gekennzeichnet durch den Gehalt an ein oder zwei 6α-Methylkortikoiden gemäß Anspruch 1 bis 11.
13. Verfahren zur Herstellung von 6α-Methylkortikoiden der allgemeinen Formel I a

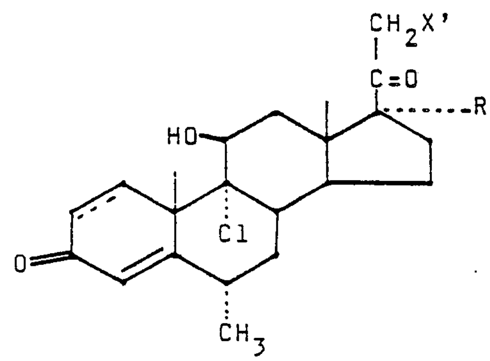

(Ia)

worin
..... eine Einfachbindung oder eine Doppelbindung bedeutet,
R eine Acyloxygruppe mit maximal 8 Kohlenstoffatomen und
X' ein Chloratom oder eine Acyloxygruppe mit maximal 8 Kohlenstoffatomen darstellen, dadurch gekennzeichnet, daß man in an sich bekannter Weise
a) ein 6α-Methylkortikoid der allgemeinen Formel II

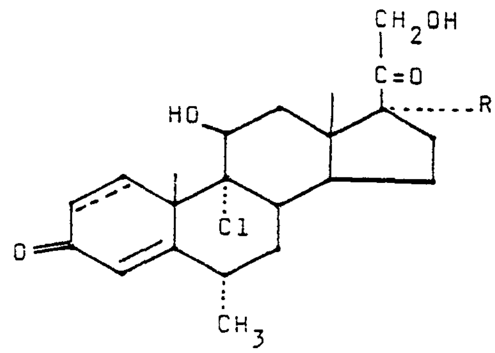

(II)

worin ..... und R die obengenannte Bedeutung besitzen, in der 21-Position verestert oder chloriert, oder
b) ein 6α-Methylkortikoid der allgemeinen Formel III

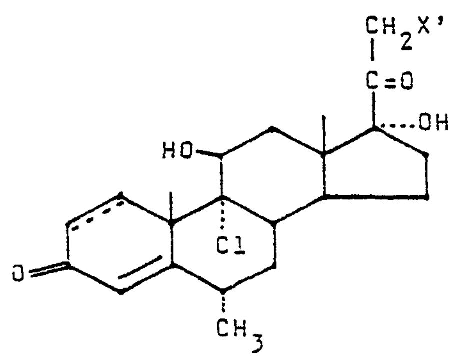

(III)

worin ..... und X' die obengenannte Bedeutung besitzen, in der 11-Position mit einer Trialkylsilylverbindung veräthert oder mit einem Derivat einer stark aciden Monocarbonsäure verestert, anschließend die 17-Position mit einem Carbonsäurechlorid oder Carbonsäureanhydrid in Gegenwart von 4-Dimethylaminopyridin acyliert und die Schutzgruppe in der 11-Position abspaltet, oder
c) an die $\Delta^{9(11)}$-Doppelbindung eines 6α-Methylkortikoids der allgemeinen Formel IV

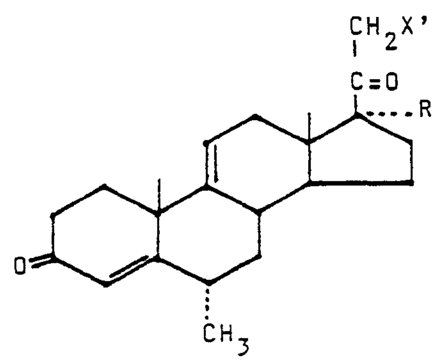

(IV)

worin ....., X' und R die obengenannte Bedeutung besitzen, unterchlorige Säure anlagert.
14. Verfahren zur Herstellung von 6α-Methylkortikoiden der allgemeinen Formel I b

(Siehe Formel Seite 8 f.)

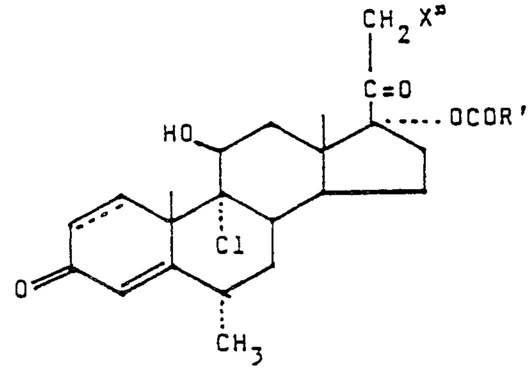

(Ib)

worin ..... eine Einfachbindung oder eine Doppelbindung und R′ eine Alkylgruppe mit maximal 7 Kohlenstoffatomen oder eine Phenylgruppe und X″ eine Hydroxygruppe oder ein Chloratom bedeuten, dadurch gekennzeichnet, daß man in an sich bekannter Weise die Ortoestergruppierung eines Kortikoids der allgemeinen Formel V

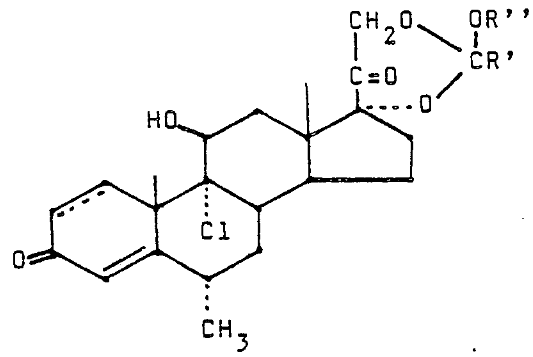

(V)

worin ..... und R′ die obengenannte Bedeutung besitzen und R″ einen Alkylrest mit 1 bis 4 Kohlenstoffatomen darstellt hydrolytisch oder mit Trimethylchlorsilan spaltet.

**Claims**

1. 6α-methyl-corticoids of the general formula I :

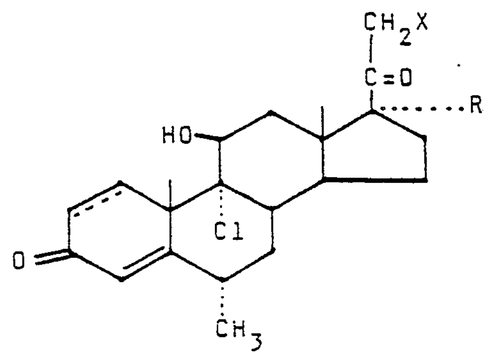

(I)

wherein

..... is a single bond or a double bond,
R is an acyloxy group with up to 8 carbon atoms and
X is a chlorine atom, a hydroxy group or an acyloxy group with upto 8 carbon atoms.

2. 17α-acetoxy-9α-chloro-11β,21-dihydroxy-6α-methyl-1,4-pregnadiene-3,20-dione.
3. 9α-chloro-11β,21-dihydroxy-6α-methyl-17α-propionyloxy-1,4-pregnadiene-3,20-dione.
4. 21-acetoxy-9α-chloro-11β-hydroxy-6α-methyl-17-α-propionyloxy-4-pregnene-3,20-dione.
5. 9α,21-dichloro-11β-hydroxy-6α-methyl-17α-propionyloxy-1,4-pregnadiene-3,20-dione.
6. 17α-benzoyloxy-9α,21-dichloro-11β-hydroxy-6α-methyl-1,4-pregnadiene-3,20-dione.
7. 9α-chloro-11β-hydroxy-6α-methyl-21-propionyloxy-17-valeryloxy-1,4-pregnadiene-3,20-dione.
8. 21-acetoxy-9α-chloro-11β-hydroxy-6α-methyl-17α-trimethylacetoxy-4-pregnene-3,20-dione.

9. 21-acetoxy-9α-chloro-11β-hydroxy-6α-methyl-17-trimethylacetoxy-1,4-pregnadiene-3,20-dione.
10 21-acetoxy-9α-chloro-11β-hydroxy-6α-methyl-17-propionyloxy-1,4-pregnadiene-3,20-dione.
11. 21-acetoxy-17-butyryloxy-9α-chloro-11β-hydroxy-6α-methyl-1,4-pregnadiene-3,20-dione.
12. Process for the preparation of 6α-methylcorticoids of the general formula Ia :

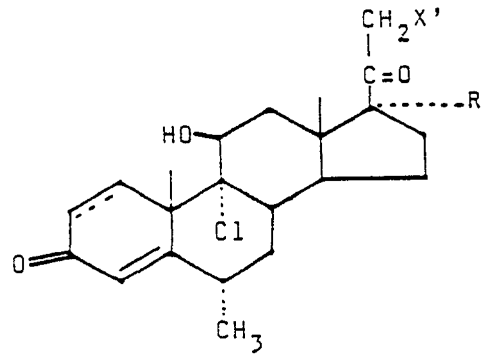

(Ia)

wherein
..... is a single bond or a double bond,
R is an acyloxy group with up to 8 carbon atoms and
X' is a chlorine atom or an acyloxy group with up to 8 carbon atoms, characterised in that in accordance with known procedures :
a) a 6α-methylcorticoid of the general formula II :

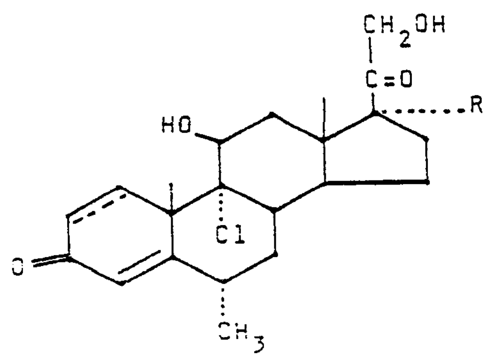

(II)

wherein ..... and R have the meanings given above, is esterified or chlorinated in the 21-position ; or
b) a 6α-methylcorticoid of the general formula III :

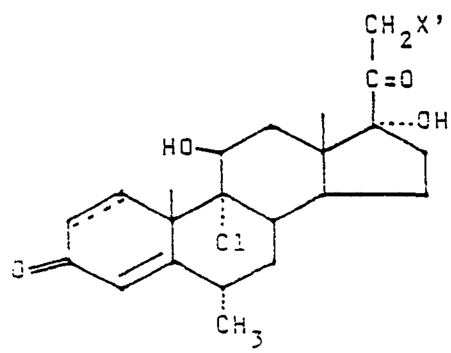

(III)

in which ..... and X' have the meanings given above, is etherified in the 11-position with a trialkylsilyl compound or esterified with a derivative of a strong monocarboxylic acid, then acylated in the 17-position with a carboxylic acid chloride or carboxylic acid anhydride in the presence of 4-dimethylaminopyridine, and the protecting group in the 11-position removed ; or
c) to the $\Delta^{9(11)}$-double bond of a 6α-methylcorticoid of the general formula IV :

(See formula page 10)

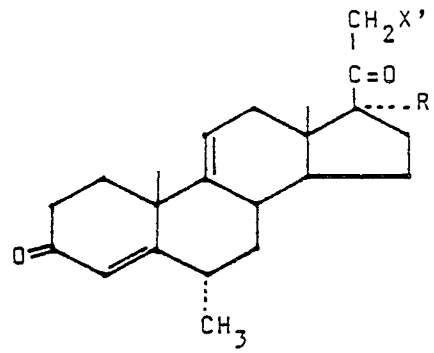

(IV)

wherein ....., X' and R have the meanings given above, is added hypochlorous acid.

14. Process for the preparation of 6α-methylcorticoids of the general formula Ib :

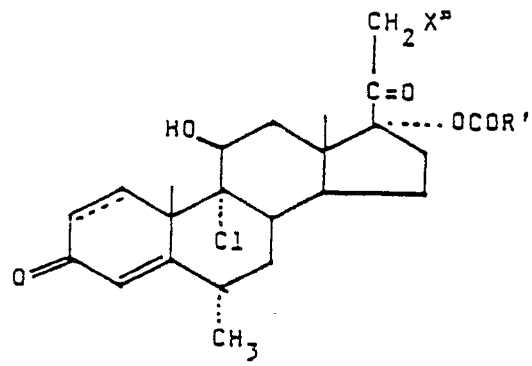

(Ib)

wherein ..... is a single bond or a double bond, R' is an alkyl group with up to 7 carbon atoms or a phenyl group, and X" is an hydroxy group or a chlorine atom, characterised in that in accordance with known procedures : the orthoester group of a corticoid of the general formula V :

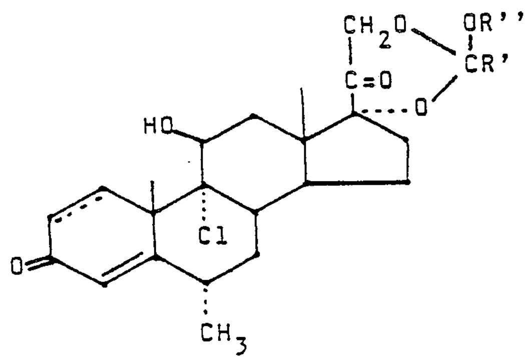

(V)

wherein ..... and R' have the meanings given above and R" is an alkyl group with 1 to 4 carbon atoms, is removed by hydrolysis or by trimethyl chlorosilane.

## Revendications

1. 6α-Méthylcorticoïdes de formule générale I ci-dessous :

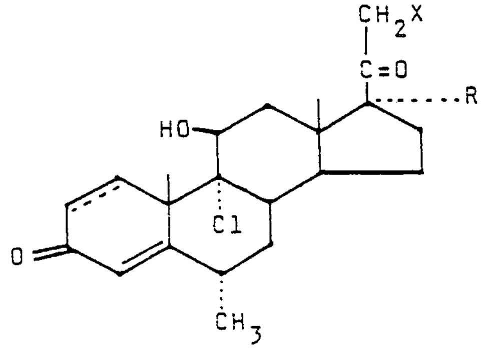

(I)

dans laquelle

..... représente une liaison simple ou double,

R un groupe acyloxy ayant au maximum 8 atomes de carbone et

X un atome de chlore, un hydroxyle ou un groupe acyloxy ayant au maximum 8 atomes de carbone.

2. 17α-Acétoxy-9α-chloro-11β,21-dihydroxy-6α-méthyl-1,4-prégnadiène-3,20-dione.

3. 9α-Chloro-11β-21-dihydroxy-6α-méthyl-17α-propionyloxy-1,4-prégnadiène-3,20-dione.

4. 21-Acétoxy-9α-chloro-11β-hydroxy-6α-méthyl-17α-propionyloxy-4-prégnène-3,20-dione.

5. 9α,21-Dichloro-11β-hydroxy-6α-méthyl-17α-propionyloxy-1,4-prégnadiène-3,20-dione.

6. 17α-Benzoyloxy-9α,21-dichloro-11β-hydroxy-6α-méthyl-1,4-prégnadiène-3,20-dione.

7. 9α-Chloro-11β-hydroxy-6α-méthyl-21-propionyloxy-17-valéryloxy-1,4-prégnadiène-3,20-dione.

8. 21-Acétoxy-9α-chloro-11β-hydroxy-6α-méthyl-17α-triméthylacétoxy-4-prégnène-3,20-dione.

9. 21-Acétoxy-9α-chloro-11β-hydroxy-6α-méthyl-17-triméthylacétoxy-1,4-prégnadiène-3,20-dione.

10. 21-Acétoxy-9α-chloro-11β-hydroxy-6α-méthyl-17-propionyloxy-1,4-prégnadiène-3,20-dione.

11. 21-Acétoxy-17-butyryloxy-9α-chloro-11β-hydroxy-6α-méthyl-1,4-prégnadiène-3,20-dione.

12. Préparation pharmaceutique caractérisée en ce qu'elle contient un ou deux 6α-méthylcorticoïdes selon les revendications 1 à 11.

13. Procédé de préparation des 6α-méthylcorticoïdes de formule générale Ia :

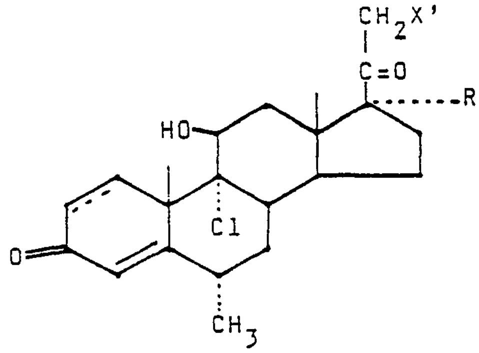

(Ia)

dans laquelle

..... représente une liaison simple ou double,

R un groupe acyloxy ayant au maximum 8 atomes de carbone et

X' un atome de chlore, un hydroxyle ou un groupe acyloxy ayant au maximum 8 atomes de carbone, procédé caractérisé en ce que, de manière connue :

a) on estérifie ou on chlore à la position 21 un 6α-méthylcorticoïde de formule II :

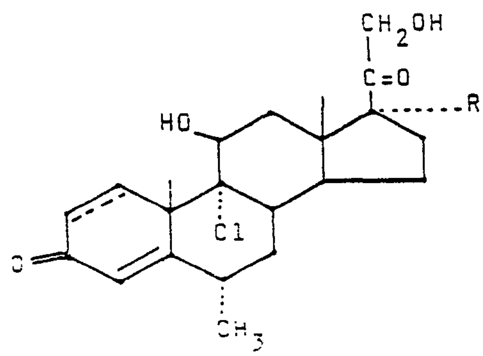

(II)

ou bien

b) on éthérifie la position 11 avec un composé trialkylsilylique un 6α-méthyl-corticoïde de formule III :

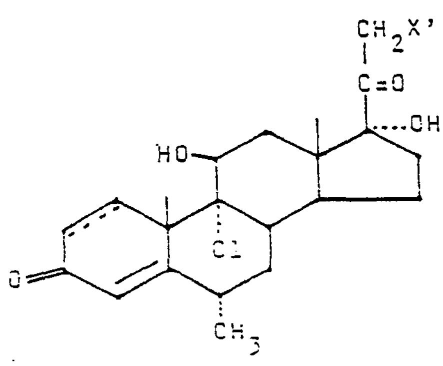

(III)

X′ étant un atome de chlore ou un groupe alcoxy ayant au maximum 8 atomes de carbone, ou l'on estérifie avec un dérivé d'un acide monocarboxylique fort, puis on acyle la position 17 avec le chlorure ou l'anhydride d'un acide carboxylique en présence de 4-diméthylaminopyridine et on élimine le groupe protecteur de la position 11,

ou bien

c) on fixe de l'acide hypochloreux sur la double liaison $\Delta^{9(11)}$ d'un 6α-méthylcorticoïde de formule IV :

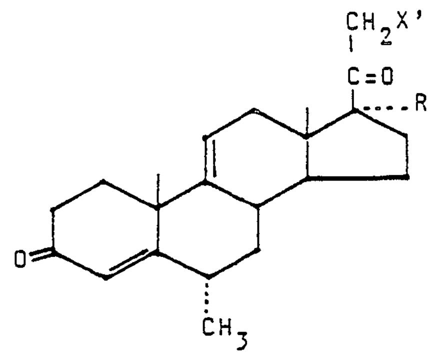

(IV)

X′ étant un atome de chlore ou un groupe alcoxy ayant au maximum 8 atomes de carbone.

14. Procédé de préparation des 6α-méthylcorticoïdes de formule générale Ib :

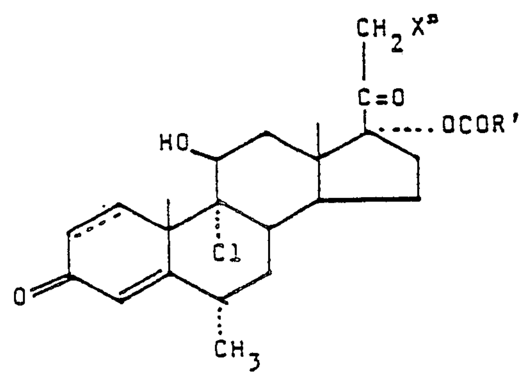

(Ib)

dans laquelle ..... représente une liaison simple ou double, R′ un alkyle ayant au maximum 7 atomes de carbone ou un groupe phényle et X″ un hydroxyle ou un atome de chlore, procédé caractérisé en ce que, de manière connue, on scinde par hydrolyse ou avec du triméthylchlorosilane le groupe orthoester d'un corticoïde de formule générale V :

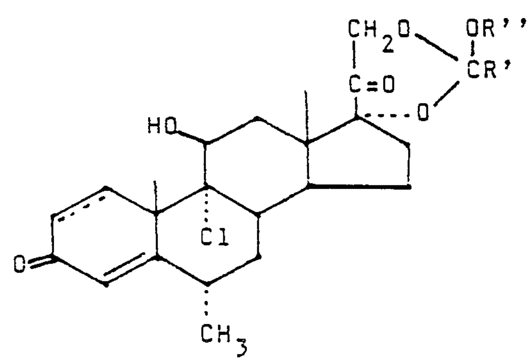

(V)

R″ étant un alkyle en $C_1$ à $C_4$.